(19) Europäisches Patentamt
European Patent Office
Office européen des brevets

(11) EP 4 792 813 A1

(12) EUROPEAN PATENT APPLICATION
published in accordance with Art. 153(4) EPC

(43) Date of publication:
19.08.2026 Bulletin 2026/34

(21) Application number: 24876581.0

(22) Date of filing: 10.10.2024

(51) International Patent Classification (IPC):
*A61K 31/454* (2006.01) *A61P 31/04* (2006.01)
*A61P 29/00* (2006.01)

(52) Cooperative Patent Classification (CPC):
A61K 31/454; A61P 29/00; A61P 31/04

(86) International application number:
PCT/CN2024/123894

(87) International publication number:
WO 2025/077769 (17.04.2025 Gazette 2025/16)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 10.10.2023 CN 202311306220

(71) Applicant: Lunan Pharmaceutical Group Corporation
Linyi, Shandong 276006 (CN)

(72) Inventors:
- ZHANG, Guimin
Shandong 276006 (CN)
- LI, Honghua
Shandong 276006 (CN)
- YAO, Jingchun
Shandong 276006 (CN)
- LU, Xiaoyan
Shandong 276006 (CN)
- MIN, Shujun
Shandong 276006 (CN)

(74) Representative: Meissner Bolte Partnerschaft mbB
Patentanwälte Rechtsanwälte
Postfach 86 06 24
81633 München (DE)

# (54) USE OF PIPERIDINE DERIVATIVE IN PREPARATION OF DRUG FOR PREVENTING AND TREATING SEPSIS AND SEPSIS-INDUCED ORGAN DAMAGE

(57) The present disclosure discloses use of a piperidine derivative or a pharmaceutically acceptable salt thereof in the preparation of a medicament for preventing and treating sepsis and sepsis-induced organ injury. Pharmacodynamic experimental studies have shown that the piperidine derivative of the present disclosure is capable of obviously reducing the levels of serum inflammatory factors in a sepsis model, effectively preventing and treating sepsis and sepsis-induced organ injury, protecting liver, kidney and heart, improving cognitive functions in patients with sepsis-associated encephalopathy, reducing a brain index and alleviating brain tissue injury, and has relatively high clinical application prospects.

Normal group

Model group

Compound I low-dose group

Compound I medium-dose group    Compound I high-dose group

FIG. 1

EP 4 792 813 A1

## Description

### TECHNICAL FIELD

The present disclosure belongs to the field of pharmaceutical technology, and relates to medical use of a piperidine derivative, in particular to use of N-(3-chlorophenyl)-1-(1-methyl-1H-indole-2-carbonyl)piperidine-4-carboxamide in the preparation of a medicament for preventing and treating sepsis and sepsis-induced organ injury.

### BACKGROUND

Sepsis is a potentially fatal systemic disease that is caused by pathogens and/or systemic inflammatory responses. It is often secondary to trauma, burns, surgical operations and the like, and is characterized by severe conditions, high incidence and high mortality. There are more than 18 million patients with severe sepsis worldwide each year, and this number is still increasing at an annual rate of 1.5% - 8.0%. Foreign epidemiological surveys show that the mortality rate of sepsis has exceeded that of myocardial infarction, making it the leading cause of death among non-cardiac patients in intensive care units.

Due to its persistent and uncontrolled inflammatory responses, sepsis results in the release of a large number of inflammatory mediators and anti-inflammatory mediators into the blood, leading to multiple organ dysfunction, lymphocyte apoptosis, immune paralysis, etc. The organ injury caused by sepsis is the main cause of poor prognosis in sepsis. The reduction in the severity of the organ injury caused by sepsis is also of great significance for the treatment of sepsis. In essence, the complications of sepsis are clinical manifestations of sepsis in various pathophysiological stages. The common complications include shock, acute lung injury/acute respiratory distress syndrome, etc. Sepsis is a systemic dysregulated inflammatory response that has profound effects on various organ systems. Except the liver and kidney, the central nervous system is a common affected site, which is often involved before other organs are affected in the early stage of the disease. Without central nervous system infection and other potential causes of brain dysfunction, diffuse brain dysfunction induced by infection is referred to as sepsis-associated encephalopathy (SAE).The study has shown that SAE may occur in up to 70% patients with sepsis. The clinical manifestations are varying degrees of brain dysfunction, ranging from mild delirium to severe coma and even death. In the acute phase of SAE, its clinical manifestation is a change in consciousness level; and in the chronic phase of SAE, its clinical manifestation is cognitive dysfunction, and even dementia.

At present, there is a lack of specific drugs to clinically treat sepsis. The treatment of sepsis is dominated by mainly symptomatic and supportive treatments, including antibiotic treatment, surgery or interventional therapy to eliminate the source of infection, and intravenous infusion to maintain adequate blood volume; administration of vasoconstrictor and/or inotropic drugs (e.g., vasopressin, norepinephrine); and supportive treatments such as mechanical ventilation, hemodialysis, and extracorporeal oxygenation. The antibacterial drug is a key to treat sepsis. However, the abuse of antibacterial drugs and the increasingly severe nosocomial infections have led to the emergence of multiple drug-resistant strains, thereby greatly reducing the therapeutic effect of antibacterial drugs. Though anti-infective treatment and organ function support techniques have made considerable progress, the fatality rate of sepsis is still up to 30% - 70%. The recommended treatment for sepsis-associated encephalopathy focuses on systemic infection management, but few therapies can effectively alleviate cognitive dysfunction. Most of the mature inhibitors targeting peripheral inflammation are not developed for the brain, which lack the ability to penetrate through the blood-brain barrier. The clinical treatments of sepsis and its associated diseases are dominated by infection control or non-specific treatment such as use of glucocorticoids, and symptomatic treatments such as prevention and treatment of organ failure and shock. These clinical treatments can alleviate sepsis symptoms and prolong survival in some patients, but their efficacies are limited.

### SUMMARY

To seek a new effective medicament for preventing and treating sepsis and sepsis-induced organ injury, inventors creatively use N-(3-chlorophenyl)-1-(1-methyl-1H-indole-2-carbonyl)piperidine-4-carboxamide (Compound I) for the treatment of sepsis. It is unexpectedly discovered that the compound possesses significant therapeutic effects and also exhibits an obvious effect on sepsis-induced organ injury. Therefore, the present disclosure provides use of Compound I or a pharmaceutically acceptable salt thereof in the preparation of a medicament for preventing or treating sepsis and sepsis-induced organ injury.

The piperidine derivative of the present disclosure is N-(3-chlorophenyl)-1-(1-methyl-1H-indole-2-carbonyl) piperidine-4-carboxamide. Its specific structural formula is as follow:

Formula I

.

Compounds I involved in the present disclosure are all N-(3-chlorophenyl)-1-(1-methyl-1H-indole-2-carbonyl) piperidine-4-carboxamide.

In the use of the present disclosure, the sepsis comprises early sepsis, severe sepsis and septic shock.

In the use of the present disclosure, the sepsis is sepsis arising under stress conditions caused by severe burns, war wounds, infections, major surgeries, severe shock, radiotherapy, chemotherapy and organ transplantation, or sepsis complicated by severe hepatitis, liver cirrhosis, gastrointestinal diseases, malnutrition, biliary tract obstruction or intestinal obstruction.

In the use of the present disclosure, the sepsis is caused by infection with microorganisms.

Further, the microorganisms include, but are not limited to, Gram-negative bacteria or Gram-positive bacteria.

In the use of the present disclosure, the sepsis-induced organ injury is septic liver injury and/or septic kidney injury.

In the use of the present disclosure, the sepsis-induced organ injury is also sepsis-associated encephalopathy.

In the use of the present disclosure, the medicament is a drug capable of improving serum biochemical parameters.

Further, the medicament is a drug capable of reducing the levels of aspartate aminotransferase (AST), alanine aminotransferase (ALT), urea (UREA) and creatinine-serum (CREA-S) in serum.

In the use of the present disclosure, the medicament is a preparation prepared from Compound I or a salt thereof as an active ingredient, together with conventional pharmaceutically acceptable additives.

Further, the pharmaceutically acceptable additives are excipients, fillers, diluents, binders, disintegrants, solubilizers, adsorbents, pH buffers, preservatives, flavoring agents, or combinations thereof.

Further, the preparation is an oral preparation, an injection or an external preparation.

More further, the oral preparation is a tablet, a pill, a powder, a granule, a capsule or a syrup; the injection is an intravenous injection, an intraperitoneal injection, an intracavitary injection, an irrigant or a lavage preparation; and the external preparation comprises a patch, an ointment or a gel.

To prepare the composition of the present disclosure into tablets, various excipients well-known in the relevant art can be widely used, including a diluent, a binder, a wetting agent, a disintegrant, a lubricant and a glidant. The diluent can be starch, dextrin, sucrose, glucose, lactose, mannitol, sorbitol, xylitol, microcrystalline cellulose, calcium sulfate, dicalcium phosphate, calcium carbonate, etc.; the wetting agent can be water, ethanol, isopropanol, etc.; the binder can be starch paste, dextrin, syrup, honey, glucose solution, microcrystalline cellulose, acacia mucilage, gelatin mucilage, sodium carboxymethyl cellulose, methyl cellulose, hydroxypropyl methyl cellulose, ethyl cellulose, acrylic resin, carbomer, polyvinylpyrrolidone, polyethylene glycol, etc.; the disintegrant can be dry starch, microcrystalline cellulose, low-substituted hydroxypropyl cellulose, crospovidone, croscarmellose sodium, sodium carboxymethyl starch, sodium bicarbonate and citric acid, polyoxyethylene sorbitan fatty acid esters, sodium lauryl sulfate, etc.; and the lubricant and the glidant can be talc, silicon dioxide, stearate, tartaric acid, liquid paraffin, polyethylene glycol, etc.

The tablet can be further prepared into a coated tablet, such as a sugar-coated tablet, a film-coated tablet, an enteric-coated tablet, or a bilayer tablet and a multilayer tablet.

The present disclosure further provides a method for treating sepsis and complications thereof, comprising administrating to a patient in need thereof an effective amount of Compound I of the present disclosure.

In the use of the present disclosure, the content of Compound I in each unit dosage form of the pharmaceutical preparation is 0.1 - 99wt%.

In some examples, the content of Compound I in each unit dosage form of the pharmaceutical preparation is 0.1 - 80wt%.

In some examples, the content of Compound I in each unit dosage form of the pharmaceutical preparation is 0.1 - 60wt%.

In some examples, the content of Compound I in each unit dosage form of the pharmaceutical preparation is 0.1 - 40wt%.

In some examples, the content of Compound I in each unit dosage form of the pharmaceutical preparation is 0.1 -

20wt%.

In some embodiments of the present disclosure, Compound I can reduce the levels of serum inflammatory factors TNF-$\alpha$ and IL-1$\beta$ in a patient with sepsis.

In some embodiments of the present disclosure, Compound I can reduce the levels of serum AST, ALT, UREA and CREA-S in a patient with sepsis.

In some embodiments of the present disclosure, Compound I can prevent and treat organ injury caused by sepsis, such as liver injury caused by sepsis and/or kidney injury caused by sepsis.

In some embodiments of the present disclosure, Compound I can improve the survival rate of a patient with sepsis.

In some embodiments, Compound I can delay or halt the progression of sepsis.

In some embodiments of the present disclosure, Compound I can alleviate the severity of sepsis and/or improve the prognosis of sepsis.

In some embodiments of the present disclosure, Compound I can reduce the brain index in a patient with sepsis-associated encephalopathy.

In some embodiments of the present disclosure, Compound I can improve the cognitive dysfunction in a patient with sepsis-associated encephalopathy.

In some embodiments of the present disclosure, Compound I can alleviate the brain tissue injury in a patient with sepsis-associated encephalopathy.

The term "pharmaceutically acceptable" used herein means that a compound, composition or carrier is suitable for administration to a subject to achieve the uses or methods described herein without undue adverse side effects.

The term "treatment" used herein and other similar synonyms include alleviating, reducing or improving the symptoms of a disease or condition, inhibiting the disease or condition, e.g., preventing the progression of the disease or condition, relieving the disease or condition, ameliorating the disease or condition, relieving the symptoms caused by the disease or condition, or halting the symptoms of the disease or condition, preventing further symptoms, and improving or preventing the underlying metabolic causes of symptoms. In addition, the term also includes prophylactic purposes. The term further includes obtaining therapeutic effects and/or prophylactic effects. The therapeutic effect refers to curing or improving the underlying disease being treated. In addition, the curing or improvement of one or more physiological symptoms associated with the underlying disease is also a therapeutic effect, for example, an improvement in the patient's condition is observed, even though the patient is still affected by the underlying disease. With respect to the prophylactic effect, the composition may be administered to a patient at risk of developing a particular disease, or a patient exhibiting one or more physiological symptoms of the disease even if the diagnosis of the disease has not yet been made.

The term "effective amount", "therapeutically effective amount" or "pharmaceutically effective amount" used herein refers to an amount of at least one active substance (such as the compound of the present application) that is sufficient to alleviate one or more symptoms of the treated diseases or symptoms after being taken. The result may be the reduction and/or alleviation of signs, symptoms or causes, or any other desired changes in a biological system. For example, the "effective amount" used for treatment is an amount of a composition comprising the compound disclosed herein required to provide clinically significant symptomatic relief on the condition. An effective amount suitable for any individual cases can be determined using a technique such as a dose-escalation trial.

The terms "subject" and "patient" used herein include humans and mammals in the present application. The mammals refer to any animal species in mammals. The examples of mammals include humans; non-human primates such as monkeys; laboratory animals such as rats, mice and guinea pigs; domestic animals such as cats, dogs, rabbits, cattle, sheep, goats, horses and pigs; and captive wild animals such as lions and tigers.

The terms not defined herein have their ordinary meanings in the art.

The present disclosure has the following benefits:

In the present disclosure, a mouse sepsis model is established by intraperitoneal injection of endotoxin lipopolysaccharide (LPS). Specifically, mice are intraperitoneally injected with Compound I, and then the therapeutic effect of Compound I on sepsis and sepsis-induced organ injury is evaluated based on the changes in serum inflammatory factor levels, serum biochemical parameters, and organ histopathology respectively. The results of animal experiments demonstrate that Compound I of the present disclosure can significantly alleviate the levels of major serum inflammatory factors TNF-$\alpha$ and IL-1$\beta$ in septic mice, reduce the levels of AST, ALT, UREA and CREA-S in serum, and effectively protect organs such as liver and kidney.

By animal experiments, the effect of the compound of the present disclosure on the survival rate of the subject is observed. The results show that the compound of the present disclosure significantly increases the survival rate of the subject, delays or halts the progression of sepsis, alleviates the severity of sepsis and/or improves the prognosis of sepsis.

In the present disclosure, a preliminary experimental study is performed on the pharmacological effect of Compound I on sepsis-associated encephalopathy. A mouse sepsis-associated encephalopathy model is established by intraperitoneal injection of endotoxin lipopolysaccharide (LPS). Behavioral alterations and histopathological changes of the brain tissue are observed in mice after intraperitoneal administration of Compound I. The results of animal experiments

indicate that Compound I can improve the cognitive dysfunction, reduce the brain index and alleviate the brain tissue injury in mice with sepsis-associated encephalopathy.

The piperidine derivative of the present disclosure has good application prospects in preventing and treating sepsis and sepsis-induced organ injury.

## BRIEF DESCRIPTION OF THE DRAWINGS

FIG. 1 is a kidney hematoxylin and eosin (HE) staining diagram of normal group, model group and Compound I group after dissection, observed under a microscope at 100× magnification.

FIG. 2 is a diagram showing the survival rate of mice with LPS-induced sepsis within 48 h in Compound I group, positive control group and control group.

FIG. 3 shows the brain index of mice in normal group, model group and each Compound I administration group.

FIG. 4 is a Nissl staining diagram of hippocampus in the brain tissue of rats in normal group, model group and each Compound I administration group, observed under a microscope at 100× magnification.

FIG. 5 shows eight-arm maze test results of mice in normal group, model group and each Compound I administration group.

FIG. 6 shows novel object recognition test results of mice in normal group, model group and each Compound I administration group.

Note: compared with the normal group, #p<0.05, and ##p<0.01; and compared with the model group, *p<0.05, and **p<0.01.

## DETAILED DESCRIPTION OF THE EMBODIMENTS

Through extensive and in-depth research, the inventors of the present disclosure provide use of Compound I in the preparation of a medicament for preventing and treating sepsis and sepsis-induced organ injury.

Next, the contents of the present disclosure will be further described in detail with reference to specific embodiments. However, it should not be understood as limiting the scope of the above subject of the present disclosure to the following examples. The following examples are used for illustrating the technical solution of the present disclosure but not limiting the scope of the present disclosure. Any modification or equivalent replacement made to the technical solution of the present disclosure by persons of ordinary skill in the art without departing from the spirit and scope of the technical solution of the present disclosure shall still fall within the protection scope of the present disclosure.

### Pharmacological example 1 Pharmacological effect of Compound I on mice with LPS-induced sepsis

1. Drugs and reagents

Compound I, prepared by the method disclosed in CN111518020A and provided by Shandong New Time Pharmaceutical Co., Ltd.

Control compound 1, with a specific structure represented by the formula below,

Cl H N O N O

Control compound 1

,

prepared by the method disclosed in CN111518020A and provided by Shandong New Time Pharmaceutical Co., Ltd.

Control compound 2, with a specific structure represented by the formula below,

Control compound 2

,

prepared by the method disclosed in CN111518020A and provided by Shandong New Time Pharmaceutical Co., Ltd.

Endotoxin (LPS), produced by Sigma.

TNF-α, IL-6 and IL-1β kits, produced by Shanghai Enzyme-linked Biotechnology Co., Ltd.

ALT, AST, UREA and CREA-S kits, produced by Mindray, Shenzhen.

Hematoxylin and Eosin Staining Kit, produced by Beyotime Biotechnology Co., Ltd.

2. Experimental animals

160 male C57BL/6J mice (6 - 8 weeks old, 18 - 22 g) were purchased from Beijing Life River Laboratory Animal Technology Co., Ltd. All animals were housed in an animal facility at room temperature (22 ± 2°C) at the relative humidity of 60 ± 5% under the condition of a 12-hour light/12-hour dark cycle, with free access to water and food.

3. Experimental method

After 1 week of acclimatization, mice were randomly divided into 8 groups: normal group, model group, positive control group, control group 1, control group 2, and compound I low-, medium- and high-dose groups, with 20 mice in each group. Among them, 10 mice were dissected 24 h later, and the remaining 10 mice were observed for 48 h to record the survival rate in each group. After the experiment was started, the normal group and model group were intraperitoneally injected with normal saline according to the body weight. The positive control group was intraperitoneally injected with 1 mg/kg dexamethasone. The Compound I low-, medium- and high-dose groups were intraperitoneally injected with 1 mg/kg, 2.5 mg/kg and 5 mg/kg compound I according to the body weight, respectively. The control group 1 was intraperitoneally injected with 5 mg/kg control compound 1 according to the body weight, and the control group 2 was intraperitoneally injected with 5 mg/kg control compound 2 according to the body weight. After 1 h, the normal group was intraperitoneally injected with normal saline according to the body weight, and the model group and each administration group were injected with 20 mg/kg LPS and then dissected 24 h later.

4. Detection index

4.1 Serum inflammatory factors

After dissection, blood was collected from the inferior venae cava of mice. The blood samples were allowed to stand at room temperature for 1 h, followed by centrifugation at 3000 r/min to obtain serum. The serum was divided into two aliquots. One aliquot was processed in accordance with the instructions of TNF-α and IL-1β ELISA kits. Each well was measured at 450 nm using a microplate reader. The OD value of each sample was determined by subtracting the absorbance of the blank control well from the measured value. A standard curve was plotted based on the concentrations and OD values of standard samples to calculate the concentrations of the samples.

4.2 Serum biochemical parameters

A series of operations were performed on the serum in accordance with the instructions of the kits to detect ALT, AST, UREA and CREA-S concentrations.

4.3 Kidney pathological sections

Mouse kidney tissue sections were stained using a hematoxylin-eosin (HE) staining method. First, the baked sections were dewaxed using xylene until the sections became transparent. The dewaxed sections were rinsed with ethanol to remove xylene and then rinsed with distilled water. The dried sections were stained with a hematoxylin staining solution for 5 - 10 min and then soaked in hydrochloric acid alcohol for 10 s. The sections were washed and then stained with eosin for 1 - 2 min and then washed with gradient ethanol. Finally, the washed sections were soaked twice in xylene for 5 min each time until the sections became transparent. The sections were dried, then mounted with a neutral resin, and observed under an optical microscope.

4.4 After 48 h, the survival rate of the remaining 10 mice in each group was observed.

5. Statistical analysis

Statistical analysis was performed on the obtained data by using SPSS22.0 software. Measurement data was expressed as $\bar{x}\pm s$. One-way ANOVA was used for comparison among multiple groups, and t-test was used for comparison between two groups. $p<0.05$ indicates statistically significant difference.

6. Experimental results and analysis

6.1 Effect of Compound I on serum inflammatory factors in mice with LPS-induced sepsis

Table 1 Serum inflammatory factor levels of mice in each group ($\bar{x}\pm s$, n = 6)

| Groups | TNF-α (pg) | IL-6 (pg) |
|---|---|---|
| Normal group | 67.26 ± 10.56 | 12.64 ± 1.17 |
| Model group | 247.4 ± 18.29## | 24.97 ± 3.08## |
| Positive control group | 79.27 ± 4.86** | 16.49 ± 1.38** |
| Control group 1 | 244.20 ± 10.95 | 24.72 ± 4.03 |
| Control group 2 | 238.20 ± 9.79 | 24.56 ± 3.56 |
| Compound I low-dose group | 127.00 ± 16.42** | 20.10 ± 3.28* |
| Compound I medium-dose group | 76.30 ± 6.77** | 17.60 ± 3.20** |
| Compound I high-dose group | 14.00 ± 2.29** | 17.04 ± 2.94** |

Note: compared with the normal group, #p<0.05, and ##p<0.01; and compared with the model group, *p<0.05, and **p<0.01.

As shown in Table 1, compared with those in the normal group, the serum TNF-α levels of mice in the model group were significantly increased ($p<0.01$); compared with those in the model group, the serum TNF-α levels of mice in the positive control group were significantly reduced ($p<0.01$), and the serum TNF-α levels of mice in the Compound I low-, medium- and high-dose groups were all significantly reduced ($p<0.01$) and dose-dependent. Compared with those in the normal group, the serum IL-6 levels of mice in the model group were significantly increased ($p<0.01$); compared with those in the model group, the serum IL-6 levels of mice in the positive control group were significantly reduced ($p<0.01$), and the serum IL-6 levels of mice in each Compound I administration group were all significantly reduced ($p<0.05$, $p<0.01$), with particularly significant effects observed in the Compound I medium- and high-dose groups ($p<0.01$). There were no significant improvements in serum inflammatory factor indices in the control group 1 and the control group 2 compared with the model group. The above-mentioned experimental results show that Compound I can reduce the levels of inflammatory factors in mice with LPS-induced sepsis, thereby improving the inflammatory symptoms of the mice.

6.2 Effect of Compound I on serum biochemical parameters of mice with LPS-induced sepsis

Table 2 Serum biochemical parameters of mice in each group ($\bar{x}\pm s$, n = 7)

| Groups | ALT (U/L) | AST (U/L) | UREA (mmol/L) | CREA-S (mmol/L) |
|---|---|---|---|---|
| Normal group | 27.34 ± 1.53 | 52.14 ± 2.34 | 10.64 ± 1.65 | 8.64 ± 0.62 |
| Model group | 86.01 ± 17.95## | 270.90 ± 33.97## | 47.80 ± 2.66## | 44.56 ± 6.28## |
| Positive control group | 58.94 ± 7.12** | 142.8 ± 21.78** | 20.69 ± 5.68** | 20.86 ± 6.46** |
| Control group 1 | 78.19 ± 10.95 | 271.1 ± 25.31 | 46.47 ± 4.58 | 45.10 ± 5.98 |
| Control group 2 | 77.76 ± 12.51 | 265.20±31.82 | 47.22 ± 3.39 | 43.99 ± 5.81 |
| Compound I low-dose group | 69.44 ± 16.93** | 183.40 ± 37.18* | 35.56 ± 8.88** | 21.07 ± 5.89** |
| Compound I medium-dose group | 67.83 ± 19.01** | 180.20 ± 54.87** | 26.02 ± 9.03** | 18.64 ± 6.72** |

(continued)

| Groups | ALT (U/L) | AST (U/L) | UREA (mmol/L) | CREA-S (mmol/L) |
|---|---|---|---|---|
| Compound I high-dose group | 59.61 ± 6.89** | 137.50 ± 26.26** | 22.69 ± 10.33** | 20.80 ± 7.59** |
| Note: compared with the normal group, #p<0.05, and ##p<0.01; and compared with the model group, *p<0.05, and **p<0.01. | | | | |

As shown in Table 2, compared with those in the normal group, the serum ALT, AST, UREA and CREA-S levels of mice in the model group were all significantly increased ($p<0.01$); and compared with those in the model group, the serum ALT, AST, UREA and CREA-S levels of mice in the positive control group were all significantly reduced ($p<0.01$).Compared with those in the model group, the serum ALT levels of mice in Compound I low-, medium- and high-dose groups were all significantly reduced ($p<0.05$, $p<0.01$), with particularly significant effects observed in the Compound I high-dose group ($p<0.01$). Compared with those in the model group, the serum AST levels of mice in Compound I low-, medium- and high-dose groups were all significantly reduced ($p<0.05$, $p<0.01$), with particularly significant effects observed in the Compound I medium- and high-dose groups ($p<0.01$). Compared with those in the model group, the serum UREA levels of mice in Compound I low-, medium- and high-dose groups were all significantly reduced ($p<0.01$), with particularly significant effects observed in the Compound I high-dose group ($p<0.01$). Compared with those in the model group, the serum CREA-S levels of mice in Compound I low-, medium- and high-dose groups were all significantly reduced ( $p<0.01$). There were no significant improvements in serum ALT, AST, UREA and CREA-S levels in the control group 1 and the control group 2 compared with the model group.ALT and AST can be clinically used for identifying whether liver injury is present. An increase in UREA and CREA-S levels is a manifestation of a kidney disease, and therefore the above-mentioned experimental results show that Compound I of the present disclosure can protect the livers and kidneys of the mice with LPS-induced sepsis, and therefore has a definite protective effect on liver and kidney injury caused by sepsis.

6.3 HE staining and scoring of mouse kidney tissues

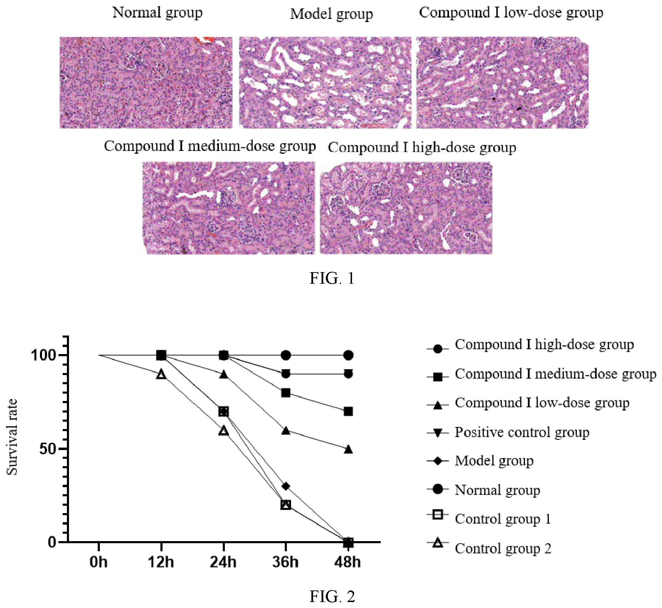

As can be seen from FIG. 1, at 24 h after intraperitoneal injection of LPS, the regular shape of renal tubules of mice in the model group was significantly changed, specifically manifested as abnormal dilation of renal tubules, atrophy of epithelial cells and lightening of cytoplasm. After administration with compound I, these changes were significantly ameliorated.

6.4 After 48 h, the survival rate of the remaining 10 mice in each group was observed, as shown in FIG. 2.

The animal experiment results show that Compound I can significantly alleviate the main serum inflammatory factors TNF-$\alpha$ and IL-1$\beta$ levels in mice with sepsis and reduce the serum AST, ALT, UREA and CREA-S levels, and therefore can effectively protect organs such as liver and kidney. The compound of the present disclosure can significantly improve the survival rate of the subject, delay or halt the progression of the sepsis, alleviate the severity of the sepsis and/or improve the prognosis of the sepsis.

**Pharmacological example 2 Effect of Compound I on brain tissue of mice with LPS-induced sepsis-associated encephalopathy**

1. Drugs and reagents

Compound I, prepared by the method disclosed in CN111518020A and provided by Shandong New Time Pharmaceutical Co., Ltd.

Endotoxin (LPS), produced by Sigma.

Nissl staining solution, produced by Beyotime.

2. Experimental animals

60 male C57BL/6J mice, weighing 18 - 22 g, were provided by Beijing HFK Bioscience Co., Ltd., with the laboratory animal use license number: SYXK (Lu) 2023-0023. The mice were housed in an SPF-grade animal room under strictly controlled environmental conditions including the temperature of 20 - 26°C, the humidity of 40 - 70% and a 12 h:12 h light-dark cycle. The mice were fed with growth and reproduction feed, with free access to food and water.

3. Experimental method

After 1 week of acclimatization, 60 male C57BL/6J mice were randomly divided into five groups: normal group,

model group, Compound I low-dose group, Compound I medium-dose group and Compound I high-dose group, with 12 mice per group.

After the experiment was started, the normal group and model group were intraperitoneally injected with normal saline according to the body weight. The Compound I low-, medium- and high-dose groups were intraperitoneally injected with 1 mg/kg, 2.5 mg/kg and 5 mg/kg Compound I according to the body weight, respectively. After 1 h, the normal group was intraperitoneally injected with a corresponding volume of normal saline according to the body weight of the mice, while the model group and each administration group were injected with 25 mg/kg LPS. The administration volume was 10 mL/kg. After 24 h, the mice were anesthetized with pentobarbital sodium. The skull was opened and the brain was harvested, rinsed with normal saline, blotted dry with filter paper, and weighed. The brain index was calculated based on the following formula:

$$\text{Brain index} = \text{brain weight/body weight} \times 100\%$$

The prepared sections were dried and dewaxed, and then stained with a Nissl staining solution for 90 min. After the sections were rinsed with running water to remove the dye, the rinsed sections were differentiated with 0.1% acetic acid until a clear background was observed under the microscope. After being rinsed with running water, the sections were counterstained for nuclei with hematoxylin in a stainer. The sections were dehydrated with anhydrous ethanol three times, followed by clarification with xylene twice, with 5 min per time. After the completion, the sections were left in a ventilated area overnight, mounted with neutral balsam, and observed under the microscope to examine the changes in structure and number of neurons among different groups.

4. Experimental results and analysis

4.1 Nissl staining results

As one of the specific structures in the neuronal cytoplasm, Nissl bodies are important sites for protein synthesis and exhibit certain basophilia, so they can be stained blue-purple by basic dyes. Nissl staining is commonly used to demonstrate changes in the structure and number of neurons in nervous system diseases such as those affecting the brain and spinal cord.

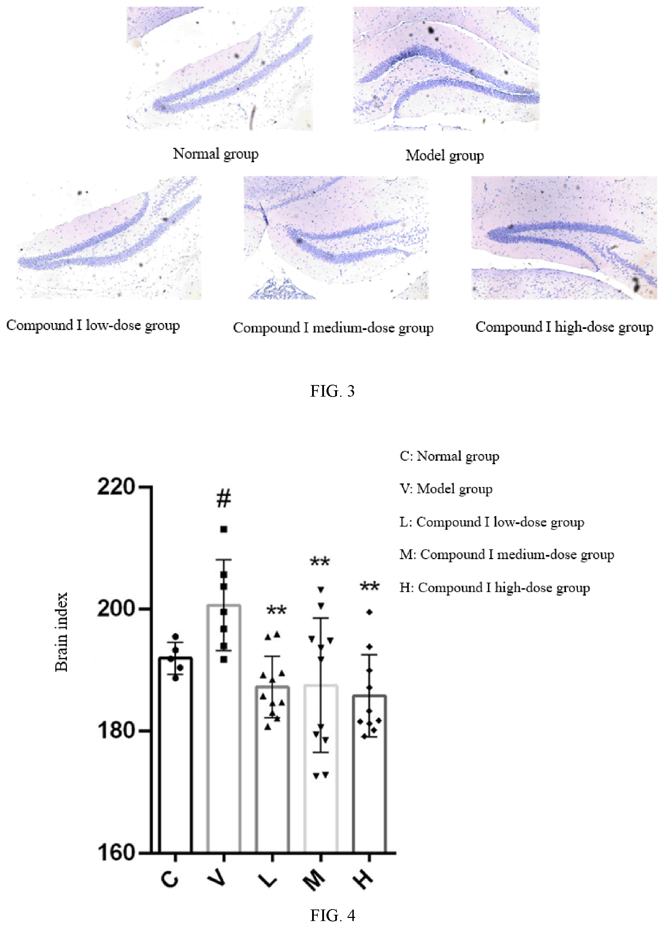

As can be seen from the Nissl staining results in FIG. 3, compared with the normal group, the model group had the injured hippocampal region of the brain tissue, neuronal pyknosis, shrunken and deformed cell bodies, and the disappearance of the Nissl bodies in the cytoplasm, indicating the impaired cellular metabolism in the hippocampal region. Compared with the model group, the Compound I low-, medium- and high-dose groups exhibited reduced neuronal pyknosis, recovered cell morphology and normal Nissl bodies in the hippocampus region of the mouse brain tissue. The above-mentioned results indicate that Compound I of the present disclosure can significantly alleviate the brain tissue injury in mice with LPS-induced sepsis-associated encephalopathy.

4.2 Brain index

As can be seen from FIG. 4, compared with the normal group, the model group exhibited a significantly increased brain index; and compared with the model group, the Compound I low-, medium- and high-dose groups exhibited a significantly reduced brain index ($p<0.01$).

**Pharmacological example 3 Behavioral effect of Compound I on mice with LPS-induced sepsis-associated encephalopathy (eight-arm maze test)**

1. Drugs and reagents

Compound I, prepared by the method disclosed in CN111518020A and provided by Shandong New Time Pharmaceutical Co., Ltd.

Endotoxin (LPS), produced by Sigma.

2. Experimental animals

60 male C57BL/6J mice, weighing 18 - 22 g, were provided by Beijing HFK Bioscience Co., Ltd., with the laboratory animal use license number: SYXK (Lu) 2023-0023. The mice were housed in an SPF-grade animal room under strictly controlled environmental conditions including the temperature of 20 - 26°C, the humidity of 40 - 70% and a 12 h:12 h

light-dark cycle. The mice were fed with growth and reproduction feed, with free access to food and water.

3. Experimental method

After 1 week of acclimatization, 60 male C57BL/6J mice were randomly divided into five groups: normal group, model group, compound I low-dose group, Compound I medium-dose group and Compound I high-dose group, with 12 mice in each group.

After the experiment was started, the normal group and model group were intraperitoneally injected with normal saline according to the body weight. The Compound I low-, medium- and high-dose groups were intraperitoneally injected with 1 mg/kg, 2.5 mg/kg and 5 mg/kg compound I, respectively. After 1 h, the normal group was intraperitoneally injected with a corresponding volume of normal saline according to the body weight of the mice, while the model group and each administration group were injected with 10 mg/kg LPS, with an administration volume of 10 mL/kg.

Behavioral tests were performed in an automated eight-arm maze. Before the experiment was started, all the mice were required to undergo acclimatization training. The food intake of the mice was restricted daily to facilitate the rapid finding and memorization of food pellets placed in the 1st, 2nd, 4th and 7th arms during the test. On day 1, an equal amount of food pellets was placed in each arm. The mice were placed in the maze and then allowed to explore freely for 300 s before acclimatization was terminated. Starting from day 2, the automatic doors were first closed, and the mice were placed in the center of the maze for 15 s. The automatic doors were then opened to release the mice for testing. The training was terminated when the time reached 300 s or the mice found the food pellets. The training was conducted for a total of five days. Working memory errors were defined as repeated entries by the mice into arms where food pellets had already been found and consumed. Reference memory errors were defined as entries into arms without food pellets. The total number of arm entries was recorded as the total arm entries. The time required for the mice to find all food pellets was defined as the test duration. On the day 7 of the experiment, a formal trial was initiated, and this step unchanged. The frequencies of working memory errors and reference memory errors were calculated based on the recorded working memory errors, reference memory errors and total arm entries.

4. Statistical analysis

Statistical analysis was performed on the obtained data by using SPSS22.0 software. Measurement data was expressed as $\bar{x}\pm s$. One-way ANOVA was used for comparison among multiple groups, and t-test was used for comparison between two groups. $p<0.05$ indicates statistically significant difference.

5. Experimental results and analysis

Working memory refers to the ability to temporarily store and process information within a short period, and its error frequency reflects short-term memory function, which is mainly associated with the prefrontal cortex. Reference memory refers to long-term stored information, and its error frequency reflects long-term memory ability; and the storage and retrieval of long-term memory mainly involve the hippocampus and parts of the cerebral cortex.

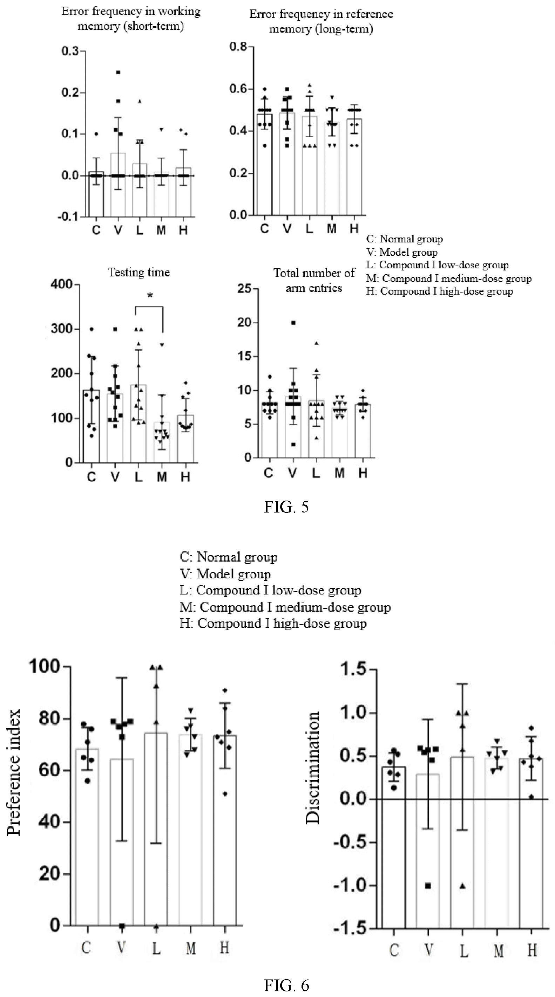

It can be seen from FIG. 5 that compared with those in the normal group, the frequencies of working memory errors and reference memory errors in the model group were both increased. Compared with those in the model group, the frequencies of working memory errors and reference memory errors in each compound I administration group were decreased, with the best effect in the Compound I medium-dose group.

Compared with those in model group, the test duration in the Compound I medium- and high-dose groups was decreased; and compared with those in the model group, the total numbers of arm entries in the Compound I medium- and high-dose groups were decreased, with the most significant reduction observed in the Compound I medium-dose group.

Based on the above experimental results, compared with the normal group, the model group showed slightly impaired cognitive function and a higher frequency of reference memory errors, indicating damage to the hippocampus or cerebral cortex. Compared with the model group, all the compound I administration groups exhibited significant improvement in cognitive function, with the most marked improvement observed in the medium- and high-dose groups.

**Pharmacological example 4 Behavioral effect (novel object recognition) of Compound I on mice with LPS-induced sepsis-associated encephalopathy**

1. Drugs and reagents

Compound I, prepared by the method disclosed in CN111518020A and provided by Shandong New Time Pharmaceutical Co., Ltd.

Endotoxin (LPS), produced by Sigma.

2. Experimental animals

60 male C57BL/6J mice, weighing 18 - 22 g, were provided by Beijing HFK Bioscience Co., Ltd., with the laboratory animal use license number: SYXK (Lu) 2023-0023. The mice were housed in an SPF-grade animal room under strictly controlled environmental conditions including the temperature of 20 - 26°C, the humidity of 40 - 70% and a 12 h:12 h light-dark cycle. The mice were fed with growth and reproduction feed, with free access to food and water.

3. Experimental method

After 1 week of acclimatization, 60 male C57BL/6J mice were randomly divided into five groups: normal group, model group, Compound I low-dose group, Compound I medium-dose group and Compound I high-dose group, with 12 mice per group.

On day 1 of the experiment, the normal group and model group were intraperitoneally injected with normal saline according to the body weight. The Compound I low-, medium- and high-dose groups were intraperitoneally injected with 1 mg/kg, 2.5 mg/kg and 5 mg/kg Compound I according to the body weight, respectively. After 1 h, the normal group was intraperitoneally injected with a corresponding volume of normal saline according to the body weight of the mice, while the model group and each administration group were injected with 7.5 mg/kg LPS. The administration volume was 10 mL/kg.

On day 2 of the experiment, the operations on day 1 were repeated. On day 3 of the experiment, the normal group and model group were intraperitoneally injected with a corresponding volume of normal saline according to the body weight of the mice. The Compound I low-, medium- and high-dose groups were intraperitoneally injected with 1 mg/kg, 2.5 mg/kg and 5 mg/kg Compound I according to the body weight, respectively. The administration volume was 10 mL/kg. After 7 days, a novel object recognition test was performed to analyze the cognitive function of the mice.

The mice were individually placed in a square arena (40 × 40 × 40 cm) and allowed to move freely for 10 min. On day 2, two identical objects, A1 and A2, were placed diagonally in the arena, and the mice were allowed to move freely for 5 min. After 120 min, the familiar object A1 and a novel object A3 were placed in the arena, and the mice were allowed to move freely for another 5 min. The time and frequency of mice contacting the objects or exploring within 2 cm of the objects (forepaws on the object, nose sniffing, or licking the object) were recorded. The preference index (PI) and discrimination index were calculated.

Preference index (PI) = [Time spent observing the novel object (A3, s)/Total time spent observing both objects (A1 and A3, s)] × 100%.

Discrimination index = (Time spent observing the novel object (A3, s) - Time spent observing the familiar object (A1, s))/(Time spent observing the novel object (A3, s) + Time spent observing the familiar object (A1, s))

4. Statistical analysis

Statistical analysis was performed on the obtained data by using SPSS22.0 software. Measurement data was expressed as $\bar{x} \pm s$. One-way ANOVA was used for comparison among multiple groups, and t-test was used for comparison between two groups. $p<0.05$ indicates statistically significant difference.

5. Experimental results and analysis

A preference index closer to 1 indicates a stronger preference for the novel object, while the preference index closer to 0 indicates a stronger preference for the familiar object, reflecting the exploratory ability of the animals. As can be seen from FIG. 6, compared with the normal group, the model group exhibited a reduced preference index, indicating that the exploratory ability of the mice in the model group was reduced; and compared with the model group, each Compound I administration group exhibited an increased preference index. Compared with the normal group, the model group exhibited a slightly reduced discrimination index, indicating that the mice in the model group had a poor cognitive ability toward familiar objects; and compared with the model group, each Compound I administration group exhibited an increased discrimination index, indicating that the mice in each Compound I administration group had a good cognitive ability toward familiar objects. All publications and patent applications cited in this application are indicative of the level of ordinary skill in the art to which this disclosure pertains. They are all incorporated herein by reference in their entirety.

The above embodiments do not limit the present disclosure in any form. For those skilled in the art, any modifications, substitutions and the like made within the scope of the technical solutions of the present disclosure based on the technical essence of the present disclosure shall still fall within the protection scope of the technical solutions of the present disclosure.

## Claims

1. Use of a piperidine derivative or a pharmaceutically acceptable salt thereof in the preparation of a medicament for preventing and treating sepsis and sepsis-induced organ injury, wherein the piperidine derivative is N-(3-chlorophenyl)-1-(1-methyl-1H-indole-2-carbonyl)piperidine-4-carboxamide.

2. The use according to claim 1, wherein the sepsis comprises early sepsis, severe sepsis and septic shock.

3. The use according to claim 1, wherein the sepsis is sepsis arising under stress conditions caused by severe burns, war wounds, infections, major surgeries, severe shock, radiotherapy, chemotherapy and organ transplantation, or sepsis complicated by severe hepatitis, liver cirrhosis, gastrointestinal diseases, malnutrition, biliary tract obstruction or intestinal obstruction.

4. The use according to claim 1, wherein the sepsis is caused by infection with microorganisms.

5. The use according to claim 4, wherein the microorganisms comprise, but are not limited to, Gram-negative bacteria or Gram-positive bacteria.

6. The use according to claim 1, wherein the sepsis-induced organ injury is septic liver injury, septic kidney injury and/or septic heart injury.

7. The use according to claim 1, wherein the sepsis-induced organ injury is sepsis-associated encephalopathy.

8. The use according to claim 1, wherein the medicament is a drug capable of improving serum biochemical parameters.

9. The use according to claim 8, wherein the medicament is a drug capable of reducing the levels of aspartate aminotransferase (AST), alanine aminotransferase (ALT), urea (UREA) and creatinine-serum (CREA-S) in serum.

10. The use according to claim 1, wherein the medicament is a preparation prepared from N-(3-chlorophenyl)-1-(1-methyl-1H-indole-2-carbonyl)piperidine-4-carboxamide or a salt thereof as an active ingredient, together with conventional pharmaceutically acceptable additives; and the pharmaceutically acceptable additives are excipients, fillers, diluents, binders, disintegrants, solubilizers, adsorbents, pH buffers, preservatives, flavoring agents, or combinations thereof.

11. The use according to claim 10, wherein the preparation is an oral preparation, an injection or an external preparation; the oral preparation is a tablet, a pill, a powder, a granule, a capsule or a syrup; the injection is an intravenous injection, an intraperitoneal injection, an intracavitary injection, an irrigant or a lavage preparation; and the external preparation comprises a patch, an ointment or a gel.

Normal group
Model group
Compound I low-dose group
Compound I medium-dose group
Compound I high-dose group

FIG. 1

Survival rate
100
50
0
0h
12h
24h
36h
48h
Compound I high-dose group
Compound I medium-dose group
Compound I low-dose group
Positive control group
Model group
Normal group
Control group 1
Control group 2

FIG. 2

Normal group
Model group
Compound I low-dose group
Compound I medium-dose group
Compound I high-dose group

FIG. 3

C: Normal group
V: Model group
L: Compound I low-dose group
M: Compound I medium-dose group
H: Compound I high-dose group
Brain index
220
200
180
160
#
**
**
**
C
V
L
M
H

FIG. 4

Error frequency in working memory (short-term)
Error frequency in reference memory (long-term)
0.3
0.2
0.1
0.0
-0.1
0.8
0.6
0.4
0.2
0.0
C V L M H
C V L M H
C: Normal group
V: Model group
L: Compound I low-dose group
M: Compound I medium-dose group
H: Compound I high-dose group
Testing time
400
300
200
100
0
*
Total number of arm entries
25
20
15
10
5
0
C V L M H
C V L M H

FIG. 5

C: Normal group
V: Model group
L: Compound I low-dose group
M: Compound I medium-dose group
H: Compound I high-dose group
Preference index
100
80
60
40
20
0
C V L M H
Discrimination
1.5
1.0
0.5
0.0
-0.5
-1.0
-1.5
C V L M H

FIG. 6

**INTERNATIONAL SEARCH REPORT**

International application No. **PCT/CN2024/123894**

**A. CLASSIFICATION OF SUBJECT MATTER**

A61K31/454(2006.01)i； A61P31/04(2006.01)i； A61P29/00(2006.01)i

According to International Patent Classification (IPC) or to both national classification and IPC

**B. FIELDS SEARCHED**

Minimum documentation searched (classification system followed by classification symbols)

IPC:A61K,A61P

Documentation searched other than minimum documentation to the extent that such documents are included in the fields searched

CNTXT, VEN, DWPI, WPABS, WPABSC, ENTXT, ENTXTC, WOTXT, USTXT, EPTXT, JPTXT, CNKI, 百度, BAIDU, 百度学术, BAIDU SCHOLAR, ISI-WEB OF SCIENCE, 必应, BING, STNext, Pubmed: 鲁南制药, 张贵民, 李红华, 姚景春, 卢小艳, 闵姝君, 脓毒, encephalopathy, SAE, 哌啶, 甲酰胺, MAGL, 单酰基甘油脂肪, Monoacylglycerol Lipase, AST, ALT, UREA, CREA, TNF, IL, 损伤, 抗炎, 炎症, 感染, 休克, 脑, 抗菌, 细菌, 微生物, injury, sepsis, damage, shock, inflammatory , inflammation, brain, antibacterial, bacterial, microbial, infection, piperidine, septicopyemia, pyemia, pyaemia, pyohemia, pyotoxin, 2473459-80-0

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | CN 111518020 A (LUNAN PHARMACEUTICAL GROUP CORP.) 11 August 2020 (2020-08-11)<br>description, page 12, embodiment 11, and page 14, embodiment 21, and paragraphs [0038], [0041], and [0055] | 1-11 |
| Y | CN 115429799 A (SHANDONG NEW TIME PHARMACEUTICAL CO., LTD.) 06 December 2022 (2022-12-06)<br>abstract, and description, paragraph [0034] | 1-11 |
| Y | CN 105503710 A (WENZHOU MEDICAL UNIVERSITY) 20 April 2016 (2016-04-20)<br>description, paragraphs [0019]-[0023], and figures 2-3 | 1-11 |
| Y | CN 112552253 A (LUNAN PHARMACEUTICAL GROUP CORP.) 26 March 2021 (2021-03-26)<br>description, paragraph [0012], and claims 1-10 | 1-11 |

☑ Further documents are listed in the continuation of Box C. ☑ See patent family annex.

* Special categories of cited documents:

- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 January 2025** | **15 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/123894

C. DOCUMENTS CONSIDERED TO BE RELEVANT

| Category* | Citation of document, with indication, where appropriate, of the relevant passages | Relevant to claim No. |
|---|---|---|
| Y | CN 114788825 A (SHANDONG NEW TIME PHARMACEUTICAL CO., LTD.) 26 July 2022 (2022-07-26)<br>abstract | 1-11 |
| A | US 2012252807 A1 (THE REGENTS OF THE UNIVERSITY OF MICHIGAN) 04 October 2012 (2012-10-04)<br>claims 1-22 | 1-11 |
| A | WO 2014053642 A1 (CONSIGLIO NAZIONALE DELLE RICERCHE) 10 April 2014 (2014-04-10)<br>claims 1-7 | 1-11 |
| Y | 康杰 等 (KANG, Jie et al.). "姜黄素对内毒素诱导的脓毒症大鼠器官功能保护及血清IL-18水平的影响 (Effect of Curcumin on Protection of Organ Function and Serum Levels of IL-18 in LPS-induced Sepsis in Rats)"<br>*临床儿科杂志 (Journal of Clinical Pediatrics),*<br>Vol. 30, No. 09, 15 September 2012 (2012-09-15),<br>pages 869-873 | 1-11 |
| Y | 刘妍 等 (LIU, Yan et al.). "脓毒症致多脏器功能损伤机制的临床研究进展 (Clinical Research Progress on the Mechanism of Multiple Organ Damage Caused by Sepsis)"<br>*锦州医科大学学报 (Journal of Liaoning Medical University),*<br>Vol. 41, No. 04, 15 August 2020 (2020-08-15),<br>pages 108-112 | 1-11 |

Form PCT/ISA/210 (second sheet) (July 2022)

**INTERNATIONAL SEARCH REPORT**
**Information on patent family members**

International application No.
**PCT/CN2024/123894**

| Patent document cited in search report | Publication date (day/month/year) | Patent family member(s) | Publication date (day/month/year) |
|---|---|---|---|
| CN 111518020 A | 11 August 2020 | None | |
| CN 115429799 A | 06 December 2022 | None | |
| CN 105503710 A | 20 April 2016 | None | |
| CN 112552253 A | 26 March 2021 | None | |
| CN 114788825 A | 26 July 2022 | None | |
| US 2012252807 A1 | 04 October 2012 | US 8846684 B2 | 30 September 2014 |
| | | WO 2012170098 A2 | 13 December 2012 |
| | | WO 2012170098 A3 | 31 January 2013 |
| WO 2014053642 A1 | 10 April 2014 | US 2015272971 A1 | 01 October 2015 |
| | | EP 2903621 A1 | 12 August 2015 |
| | | EP 2903621 B1 | 26 December 2018 |
| | | US 9351983 B2 | 31 May 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 111518020 A